# EUROPEAN PATENT SPECIFICATION

(11) **EP 3 474 744 B1**
(45) Date of publication and mention of the grant of the patent: **16.12.2020**
(21) Application number: 17722288.2
(22) Date of filing: 25.04.2017
(51) Int. Cl.: A61B 5/042, A61B 5/00, A61B 5/06

(54) **SYSTEM FOR ELECTROPHYSIOLOGY PROCEDURES**
SYSTEM FÜR ELEKTROPHYSIOLOGISCHE UNTERSUCHUNGEN
SYSTÈME DE PROCÉDURES ÉLECTROPHYSIOLOGIQUES

(30) Priority: 22.06.2016 US 201662353252 P
(43) Date of publication of application: 01.05.2019
(73) Proprietor: St. Jude Medical, Cardiology Division, Inc., St. Paul, MN 55117 (US)
(72) Inventor: MARKOVITZ, Craig, Mahtomedi 55115 MN (US); QU, Fujian, San Jose California 95118 (US); LI, Wenwen, San Jose California 95131 (US); MCSPADDEN, Luke, Los Angeles California 90026 (US)
(74) Representative: Kramer Barske Schmidtchen Patentanwälte PartG mbB
(86) International application number: PCT/US2017/029428
(87) International publication number: WO 2017/222634

(56) References cited:
- US-A1- 2008 221 643
- US-A1- 2009 253 976
- US-A1- 2016 106 336
- US-B1- 6 240 307

## Description

### CROSS-REFERENCE TO RELATED APPLICATIONS

### BACKGROUND

The instant disclosure relates to electrophysiology procedures, such as cardiac diagnostic (*e.g.*, mapping) and therapeutic (*e.g.*, ablation) procedures. In particular, the instant disclosure relates to systems, apparatuses, and methods for the creation of anatomical models and for conducting electrophysiology studies.

It is desirable to create anatomical models in connection with various medical procedures. For example, cardiac models are often created in connection with cardiac electrophysiology studies. Those of ordinary skill in the art will be familiar with numerous ways to acquire and display cardiac models for use in cardiac electrophysiology studies (*e.g*., using a roving catheter to gather a cloud of geometry points to which a surface is fit).

Once a cardiac model is created, that model can be used, for example, to display one or more electrophysiology maps. In general, electrophysiology maps are created from a plurality of electrophysiology data points, each of which includes both measured electrophysiology data and location data, allowing the measured electrophysiology information to be associated with a particular location in space (that is, allowing the measured electrophysiology information to be interpreted as indicative of electrical activity at a point on the patient's heart), and therefore displayed at the proper location on the cardiac model.

In certain extant approaches to non-contact electrophysiological mapping, a non-contact, multi-electrode mapping catheter is first introduced into the heart. Once the mapping catheter is placed, a roving catheter is introduced in order to create the cardiac model. The presence of the mapping catheter, however, can complicate the creation of the cardiac model, for example by impeding access by the roving catheter to portions of the heart. Moreover, the cardiac model can be invalidated if the mapping catheter moves during creation of the cardiac model.

US 2016/106336 A1 and US 2008/221643 A1 relate to anatomical mapping and localization of an object, wherein a catheter comprising several electrodes is used which are adapted for acquiring data for generating a model and EP data.

US 6,240,307 B1 relates to a technique for forming a two-dimensional subsurface map at a particular location in an endocardial wall by using two catheters simultaneously present in the patient's heart.

US 2009/0253976 A1 relates to determining the position of an object, in particular of one or more catheters in a patient's heart cavity by using two catheters simultaneously present within the patient's heart.

### BRIEF SUMMARY

Disclosed herein is a method of performing a cardiac electrophysiology procedure, including the steps of: inserting a catheter including at least one magnetic localization sensor into a patient's heart; generating a cardiac model using the catheter, wherein the cardiac model includes magnetic localization data measured using the at least one magnetic localization sensor; removing the catheter from the patient's heart; and, after removing the catheter from the patient's heart: inserting an electrophysiology catheter including at least one magnetic localization sensor into the patient's heart; placing the electrophysiology catheter in an initial location within the patient's heart; localizing the electrophysiology catheter within a coordinate system common to the cardiac model using the at least one magnetic localization sensor of the electrophysiology catheter; and performing the electrophysiology procedure using the electrophysiology catheter. The at least one magnetic localization sensor of the electrophysiology catheter can be positioned on a shaft of the electrophysiology catheter.

The electrophysiology procedure can be a diagnostic procedure, such as an electrophysiology mapping procedure, a therapeutic procedure, such as an ablation procedure, or a combination of both.

The electrophysiology device can be a multi-electrode electrophysiology mapping catheter, such as a non-contact electrophysiology mapping catheter.

In exemplary embodiments, the method can also include: monitoring for movement of the electrophysiology catheter from the initial location to a subsequent location; and re-localizing the electrophysiology catheter within the coordinate system common to the cardiac model using the at least one magnetic localization sensor of the electrophysiology catheter.

It is also contemplated that localizing the electrophysiology catheter within the coordinate system common to the cardiac model using the at least one magnetic localization sensor of the electrophysiology catheter can include determining a rotation angle of the at least one magnetic localization sensor of the electrophysiology catheter. For example, the electrophysiology catheter can include at least one radiopaque marker that is used to determine the rotation angle of the at least one magnetic localization sensor of the electrophysiology catheter. As another example, the rotation angle of the at least one magnetic localization sensor of the electrophysiology catheter can be determined using multiple electrodes on an electrophysiology mapping catheter.

According to another embodiment of the disclosure, a method of performing an electrophysiology procedure includes: generating a model of an anatomical region using a probe, wherein the probe includes at least one magnetic localization sensor, and wherein the model includes magnetic localization data measured using the at least one magnetic localization sensor; placing an electrophysiology device within the anatomical region, wherein the electrophysiology device includes at least one magnetic localization sensor; localizing the electrophysiology device within a coordinate system common to the model using the at least one magnetic localization sensor of the electrophysiology device; and performing the electrophysiology procedure using the electrophysiology device.

The method can include compensating for movement of the electrophysiology device during the electrophysiology procedure using the at least one magnetic localization sensor of the electrophysiology device. For example, the localizing step can be repeated if and when the electrophysiology device moves.

The electrophysiology procedure can include a diagnostic procedure and/or a therapeutic procedure.

The electrophysiology device can include a plurality of electrodes, and localizing the electrophysiology device within the coordinate system common to the model using the at least one magnetic localization sensor of the electrophysiology device can include using the plurality of electrodes to determine a rotation angle of the electrophysiology device.

Alternatively, the electrophysiology device can include a radiopaque marker, and localizing the electrophysiology device within the coordinate system common to the model using the at least one magnetic localization sensor of the electrophysiology device can include using the radiopaque marker to determine a rotation angle of the electrophysiology device.

In still other embodiments of the instant disclosure, a method of performing an electrophysiology procedure includes: inserting a probe carrying at least one magnetic localization sensor into an anatomical region; generating a model of the anatomical region by measuring a plurality of positions of the at least one magnetic localization sensor as it moves through the anatomical region; removing the probe from the anatomical region; and, after removing the probe from the anatomical region: positioning an electrophysiology device including at least one magnetic localization sensor at an initial position within the anatomical region; localizing the initial position of the electrophysiology device within a coordinate system common to the model of the anatomical region using the at least one magnetic localization sensor of the electrophysiology device; and performing the electrophysiology procedure using the electrophysiology device.

The method can include: monitoring for movement of the electrophysiology device from the initial position to a subsequent position; and localizing the subsequent position of the electrophysiology device within the coordinate system common to the model of the anatomical region using the at least one magnetic localization sensor of the electrophysiology device.

It is also contemplated that localizing the initial position of the electrophysiology device within the coordinate system common to the model of the anatomical region using the at least one magnetic localization sensor of the electrophysiology device can include determining a rotation angle of the electrophysiology device.

The foregoing and other aspects, features, details, utilities, and advantages of the present invention will be apparent from reading the following description and claims, and from reviewing the accompanying drawings.

### BRIEF DESCRIPTION OF THE DRAWINGS

Figure 1 is a schematic diagram of a localization system, such as may be used in an electrophysiology procedure.
Figure 2 depicts an exemplary catheter, such as may be used to create a cardiac geometry as described herein.
Figure 3 depicts an exemplary electrophysiology catheter, such as may be used to carry out an electrophysiology procedure as disclosed herein.
Figure 4 is a flowchart of representative steps that can be carried out in the performance of an electrophysiology procedure as disclosed herein.

### DETAILED DESCRIPTION

The present disclosure provides methods, apparatuses, and systems for the creation of anatomical models in connection with electrophysiology studies. For purposes of illustration, exemplary embodiments will be described in detail herein in the context of a cardiac electrophysiology mapping procedure. It is contemplated, however, that the methods, apparatuses, and systems described herein can be utilized in other contexts including, without limitation, cardiac ablation.

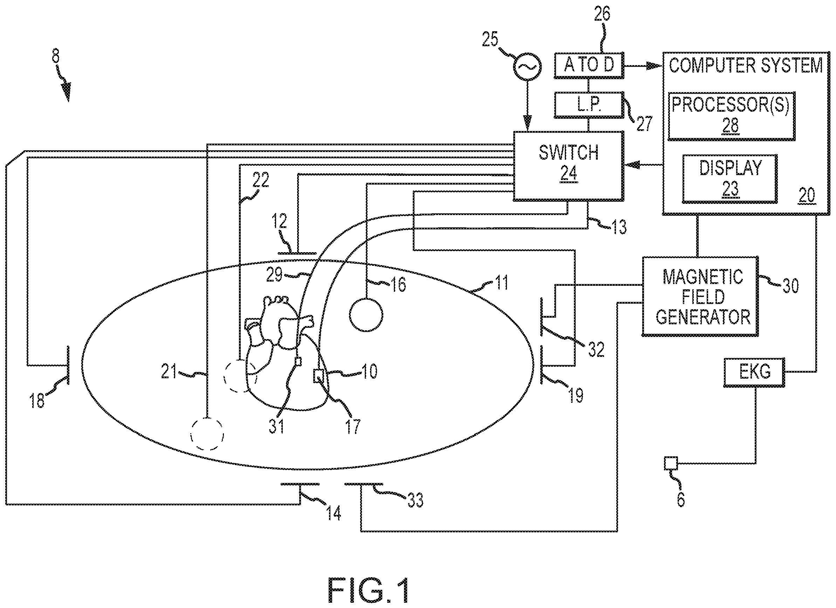

Figure 1 shows a schematic diagram of a system 8 for conducting cardiac electrophysiology studies by navigating and localizing one or more medical devices (e.g., one or more catheters 13), creating one or more cardiac models, measuring electrical activity occurring in a heart 10 of a patient 11 (depicted schematically as an oval), and/or mapping and displaying the measured electrical activity and/or information related to or representative of the measured electrical activity on the cardiac model(s). System 8 can also be used to deliver therapy (e.g., ablation) to heart 10.

Thus, system 8 can determine the location, and in some aspects the orientation, of objects, typically within a three-dimensional space, and express those locations as position information determined relative to at least one reference (e.g., belly patch 21 and/or fixed reference 31). This data can, in turn, be used to create a model of the patient's heart 10 using one or more roving localization elements, as will be familiar to those of ordinary skill in the art.

System 8 can also be used to measure electrophysiology data at a plurality of points along a cardiac surface and store the measured data in association with location information for each measurement point at which the electrophysiology data was measured. These electrophysiology data points can be used to create one or more electrophysiology maps of the patient's heart 10 according to known techniques.

As depicted in Figure 1 and described herein, system 8 is a hybrid system that incorporates both impedance-based and magnetic-based localization capabilities. In some embodiments, system 8 includes the EnSite™ Velocity™ cardiac mapping and visualization system of St. Jude Medical, Inc., which generates electrical fields, or another localization system that relies upon electrical localization fields. Other localization systems, may be used in connection with the present teachings, including, without limitation, the CARTO navigation and location system of Biosense Webster, Inc., the AURORA® system of Northern Digital Inc., Sterotaxis' NIOBE® Magnetic Navigation System, as well as MediGuide™ Technology and the EnSite Precision™ system, both from St. Jude Medical, Inc. Insofar as the ordinarily skilled artisan will appreciate the basic operation of such localization systems, they are only described herein to the extent necessary to understand the instant disclosure.

Computer 20 may comprise a conventional general-purpose computer, a special-purpose computer, a distributed computer, or any other type of computer. The computer 20 may comprise one or more processors 28, such as a single central processing unit (CPU), or a plurality of processing units, commonly referred to as a parallel processing environment, which may execute instructions to practice the various aspects disclosed herein.

For simplicity of illustration, the patient 11 is depicted schematically as an oval. In the embodiment shown in Figure 1, three sets of surface electrodes (e.g., patch electrodes 12, 14, 16, 18, 19, and 22) are shown coupled to a current source 25.

Figure 1 also depicts a magnetic source 30, which is coupled to magnetic field generators. In the interest of clarity, only two magnetic field generators 32 and 33 are depicted in Figure 1, but it should be understood that additional magnetic field generators (e.g., a total of six magnetic field generators, defining three generally orthogonal axes analogous to those defined by patch electrodes 12, 14, 16, 18, 19, and 22) can be used without departing from the scope of the present teachings.

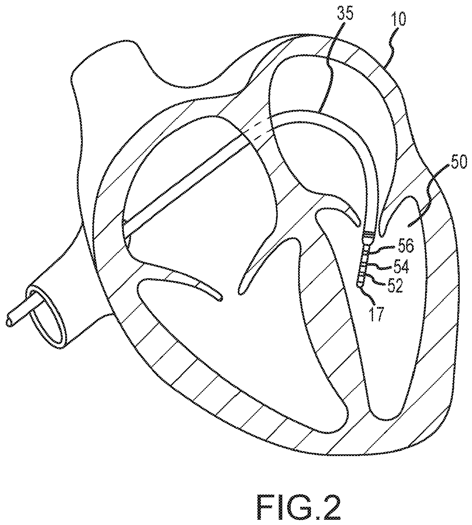

As mentioned above, system 8 can be used to create cardiac models, for example using a representative catheter 13 having at least one localization sensor. It should be understood that catheter 13 (or multiple such catheters) are typically introduced into the heart and/or vasculature of the patient via one or more introducers and using familiar procedures. For purposes of this disclosure, a segment of an exemplary catheter 13 is shown in Figure 2. In Figure 2, catheter 13 extends into the left ventricle 50 of the patient's heart 10 through a transseptal sheath 35. The use of a transseptal approach to the left ventricle is well known and need not be further described herein. Catheter 13 can also be introduced into the heart 10 in any other suitable manner.

Figure 2 also depicts multiple elements 17, 52, 54, 56 carried by catheter 13. According to aspects of the instant disclosure, at least one of elements 17, 52, 54, and 56 is a magnetic sensor that allows localization of catheter 13 within a magnetic localization field, such as may be generated by magnetic source 30 operating in conjunction with magnetic field generators 32, 33. Similarly, at least one of elements 17, 52, 54, 56 is an electrode that allows localization of catheter 13 within an impedance-based localization field, such as may be generated by current source 25 operating in conjunction with patch electrodes 12, 14, 16, 18, 19, 22.

In any event, those of ordinary skill in the art will be familiar with the use of such a catheter to create a cardiac model from a plurality of geometry points collected as catheter 13 moves through a heart chamber. As described in detail below, in embodiments of the disclosure, magnetic localizations of catheter 13 are used to generate the plurality of geometry points and the resultant cardiac model.

System 8 can also be used to create one or more electrophysiology maps. In general, electrophysiology maps are created from a plurality of electrophysiology data points, each of which includes both measured electrophysiology data and location data, allowing the measured electrophysiology information to be associated with a particular location in space (that is, allowing the measured electrophysiology information to be interpreted as indicative of electrical activity at a point on the patient's heart 10).

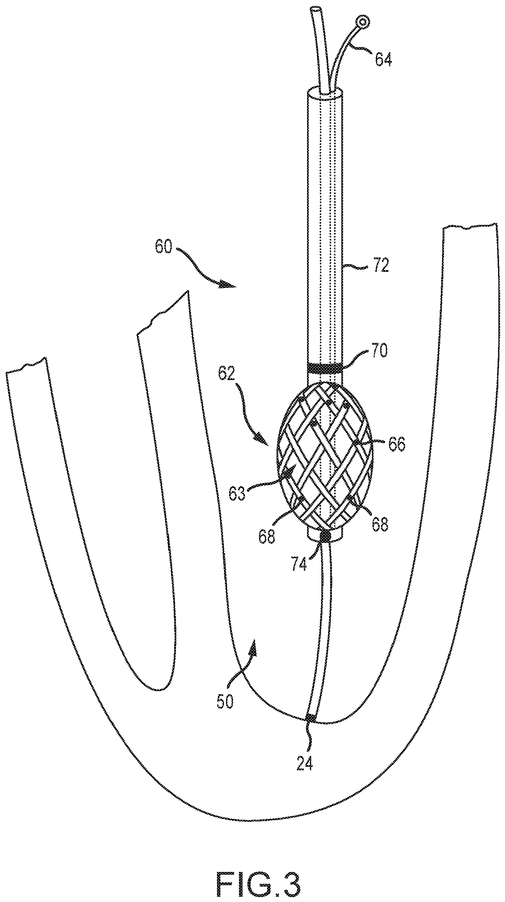

Figure 3 depicts a representative electrophysiology catheter 60, such as the EnSite™ Array™ non-contact mapping catheter of St. Jude Medical, Inc., placed into left ventricle 50 to carry out an electrophysiology procedure (e.g., an electrophysiology mapping procedure) according to the teachings herein. Electrophysiology catheter 60 includes an electrode array 62 and an inflatable balloon 63 that can be expanded, such as through the use of a stylet 64 or via inflation of balloon 63 interior to array 62, to place electrode array 62 into a stable and reproducible geometric shape.

Electrode array 62 includes a braid of insulated wires 66. The insulation can be removed from wires 66 at predetermined locations to form a plurality of electrodes 68. Additional details of representative electrophysiology catheter 60 are described in United States patent no. 6,240,307.

According to aspects of the disclosure, electrophysiology catheter 60 also includes a magnetic localization sensor 70, which allows electrophysiology catheter 60 to be magnetically localized by magnetic source 30 operating in conjunction with magnetic field generators 32, 33. In embodiments, magnetic localization sensor 70 is positioned on shaft 72 of electrophysiology catheter 60 near electrode array 62. In the embodiment shown in Figure 3, magnetic localization sensor 70 is embedded within shaft 72 at a location proximal of electrode array 62. In other embodiments, magnetic localization sensor 70 can be positioned at other locations along the length of shaft 72. For example, magnetic localization sensor 70 can be embedded within a portion of shaft 72 that is co-radial with balloon 63 such that sensor 70 is centrally located relative to array 62.

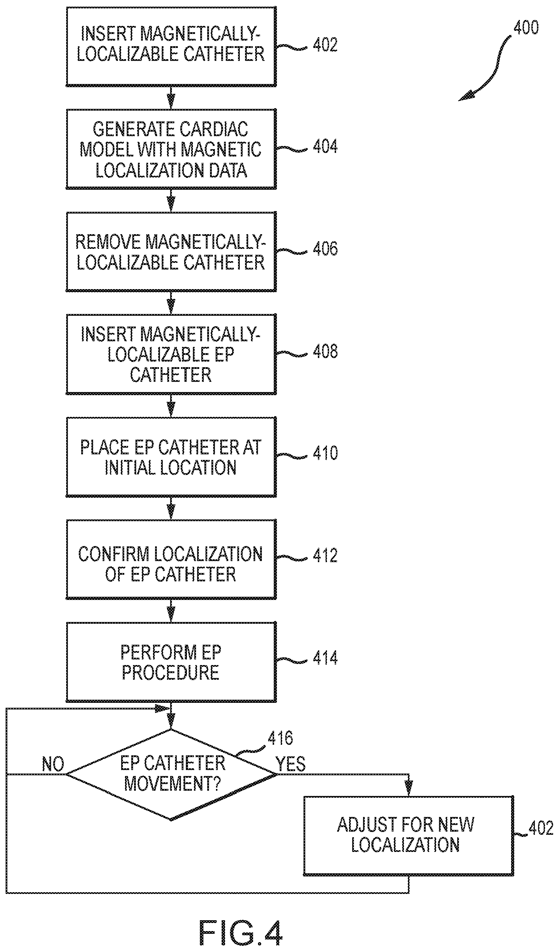

Various aspects of the instant disclosure will now be described with reference to Figure 4, which is a flowchart depicting representative steps of an exemplary method 400 for carrying out a cardiac electrophysiology procedure according to aspects of the instant disclosure. In some embodiments, for example, Figure 4 may represent several exemplary steps that can be carried out by the computer 20 of Figure 1 (*e.g.*, by one or more processors 28 executing one or more specialized modules as further described below).

It should be understood that the teachings herein can be software- and/or hardware-implemented, and that they may be executed on a single CPU, which may have one or more threads, or distributed across multiple CPUs, each of which may have one or more threads, in a parallel processing environment.

In block 402 of Figure 4, a magnetically-localizable catheter (that is, a catheter including at least one magnetic localization sensor) is inserted into a patient's heart. For example, as described above, catheter 13, carrying one or more magnetic localization sensors as shown in Figure 2 (*e.g.*, one or more of 17, 52, 54, 56), can be navigated into the patient's ventricle 50 via a transseptal introducer 35.

In block 404, the magnetically-localizable catheter is used to generate a cardiac model. For example, as described above, catheter 13 can be moved through ventricle 50 in order to collect a cloud of geometry points by periodically detecting the position of the magnetic localization sensor(s) within a magnetic localization field generated by magnetic source 30 in conjunction with magnetic field generators 32, 33. Various approaches are known for creating a three-dimensional model of the cardiac geometry from such a cloud of geometry points. The resultant model includes magnetic localization data measured using the magnetic sensor(s) on catheter 13.

In block 406, the magnetically-localizable catheter is removed from the patient's heart. For example, catheter 13 can be withdrawn back through transseptal introducer 35. Introducer 35, on the other hand, can be left in place for further steps in the electrophysiology procedure as described below.

In block 408, electrophysiology catheter 60 is inserted into the patient's heart, for example as shown in Figure 3. As described above, electrophysiology catheter 60 includes at least one magnetic localization sensor 70 such that it is magnetically localizable. This enables electrophysiology catheter 60 to be localized within the cardiac model created in block 404, as described in further detail below.

Electrophysiology catheter 60 is placed in an initial location and prepared to collect electrophysiology data points in block 410. The precise sequence of events encompassed by block 410 may differ from one electrophysiology catheter to another. In the case of the EnSite™ Array™ catheter, for example, once electrophysiology catheter 60 is positioned, electrode array 62 can be expanded, which positions electrodes 68 to record electrical activity on the surface of the heart.

In block 412, electrophysiology catheter 60 is localized within the cardiac model created in block 404 using at least the localization of the at least one magnetic localization sensor 70 on electrophysiology catheter 60 and the magnetic localization data in the cardiac model. For example, one or more fiducial points (*e.g.*, the left ventricle apex) can be identified, and the localization thereof using both the at least one magnetic sensor 70 of electrophysiology catheter 60 and the localization data in the cardiac model can be used to confirm the initial localization of electrophysiology catheter 60 within the cardiac model.

The initial localization of electrophysiology catheter 60 in block 412 can also include determining a rotation angle of electrophysiology catheter 60. More particularly, block 412 can include determining a rotation angle of the at least one magnetic localization sensor 70 carried by electrophysiology catheter 60. In embodiments of the disclosure, electrophysiology catheter 60 includes a radiopaque marker 74, and the position of radiopaque marker 74, as determined using fluoroscopy, is used to determine the rotation angle of electrophysiology catheter 60.

In other embodiments of the disclosure, such as embodiments that utilize a multi-electrode electrophysiology catheter 60, an electric field created by the electrodes can be used to determine the rotation angle of electrophysiology catheter 60.

In still other embodiments, magnetic sensor 70 can be a six-degree-of-freedom magnetic sensor that can be used independently to determine the rotation angle of electrophysiology catheter 60.

In further embodiments, two magnetic sensors 70 can be used in conjunction to determine the rotation angle of electrophysiology catheter 60.

Once electrophysiology catheter 60 has been localized within the cardiac model, including any determination of the rotation angle of electrophysiology catheter 60, the electrophysiology procedure can be carried out in block 414. For example, electrophysiology catheter 60 can be used to perform a diagnostic procedure, such as electrophysiological mapping (*e.g*., calculating electrical signals from raw signals sensed by electrophysiology catheter 60 and, in embodiments, displaying the calculated electrical signals over the cardiac model) and/or to perform a therapeutic procedure, such as an ablation procedure.

In contrast to many extant electrophysiology procedures, which are carried out with multiple catheters in the heart, the electrophysiology procedure described above is carried out with only a single catheter (namely, electrophysiology catheter 60) within the heart chamber. This minimizes the risk that electrophysiology catheter 60 will move from its initial location during the electrophysiology procedure.

Nonetheless, it should be understood that electrophysiology catheter 60 may be moved, either deliberately or accidentally, during the electrophysiology procedure. According to aspects of the disclosure, therefore, block 416 monitors for movement of electrophysiology catheter 60, for example from the initial location (block 410) to a subsequent location.

If movement is detected, block 418 can adjust (*e.g*., compensate) for the new localization of electrophysiology catheter 60, thereby minimizing or eliminating the invalidation of previously collected data and ensuring that the electrophysiology procedure can continue substantially uninterrupted.

Although several embodiments of this invention have been described above with a certain degree of particularity, those skilled in the art could make numerous alterations to the disclosed embodiments without departing from the scope of this invention.

For example, in the embodiments described above a first magnetically-localizable catheter (*e.g*., catheter 13) is used to generate the geometry, and a second magnetically-localizable catheter (*e.g*., catheter 60) is used to carry out the electrophysiology procedure. In other embodiments, however, a single magnetically-localizable catheter (*e.g*., catheter 60) can be used both to generate the geometry and to carry out the electrophysiology procedure.

All directional references (e.g., upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other.

It is intended that all matter contained in the above description or shown in the accompanying drawings shall be interpreted as illustrative only and not limiting.

## Claims

1. A system for performing a cardiac electrophysiology procedure, comprising:
a catheter (13) including at least one magnetic localization sensor (70);
an electrophysiology catheter (60) including at least one magnetic localization sensor (70); and
one or more processors (28) adapted for:
generating a cardiac model using the catheter (13) inserted within the patient's heart, wherein the cardiac model comprises magnetic localization data measured using the at least one magnetic localization sensor (70); and
localizing the electrophysiology catheter (60) which is inserted into the patient's heart after the catheter (13) has been removed from the patient's heart at an initial location within a coordinate system common to the cardiac model using the at least one magnetic localization sensor (70) of the electrophysiology catheter (60) for performing the electrophysiology procedure using the electrophysiology catheter (60).

2. The system according to claim 1, wherein the electrophysiology procedure comprises an electrophysiology mapping procedure.

3. The system according to claim 1, wherein the electrophysiology catheter (60) comprises a multi-electrode electrophysiology mapping catheter.

4. The system according to claim 3, wherein the electrophysiology catheter (60) comprises a non-contact electrophysiology mapping catheter.

5. The system according to claim 1, wherein the electrophysiology procedure comprises an ablation procedure.

6. The system according to claim 1, wherein the one or more processors (28) is further adapted for:
monitoring for movement of the electrophysiology catheter (60) from the initial location to a subsequent location; and
re-localizing the electrophysiology catheter (60) within the coordinate system common to the cardiac model using the at least one magnetic localization sensor (70) of the electrophysiology catheter (60).

7. The system according to claim 1, wherein localizing the electrophysiology catheter (60) within a coordinate system common to the cardiac model using the at least one magnetic localization sensor (70) of the electrophysiology catheter (60) further comprises determining a rotation angle of the at least one magnetic localization sensor (70) of the electrophysiology catheter (60).

8. The system according to claim 7, wherein the electrophysiology catheter (60) comprises at least one radiopaque marker (74), and wherein determining the rotation angle of the at least one magnetic localization sensor (70) of the electrophysiology catheter (60) comprises using the at least one radiopaque marker (74) to determine the rotation angle of the at least one magnetic localization sensor (70) of the electrophysiology catheter (60).

9. The system according to claim 7, wherein the electrophysiology catheter (60) comprises a multi-electrode electrophysiology mapping catheter, and wherein determining the rotation angle of the at least one magnetic localization sensor (70) of the electrophysiology catheter (60) comprises using the multiple electrodes of the electrophysiology mapping catheter to determine the rotation angle of the at least one magnetic localization sensor (70) of the electrophysiology catheter (60).

10. The system according to claim 1, wherein the at least one magnetic localization sensor (70) of the electrophysiology catheter (60) is positioned on a shaft of the electrophysiology catheter (60).

11. The system according to claim 1, wherein the one or more processors (28) is further adapted for compensating for movement of the electrophysiology catheter (60) during the electrophysiology procedure using the at least one magnetic localization sensor (70) of the electrophysiology catheter (60).

12. The system according to claim 1, wherein the electrophysiology procedure comprises a diagnostic procedure.

13. The system according to claim 1, wherein the electrophysiology procedure comprises a therapeutic procedure.

## Patentansprüche

1. System zum Durchführen einer kardialen elektrophysiologischen Prozedur, mit:
einem Katheter (13), der mindestens einen magnetischen Lokalisierungssensor (70) aufweist;
einem elektrophysiologischen Katheter (60), der mindestens einen magnetischen Lokalisierungssensor (70) aufweist; und
einem oder mehreren Prozessoren (28), die angepasst sind zum:
Erzeugen eines Herzmodells, indem der Katheter (13) verwendet wird, der in das Herz eines Patienten eingeführt ist, wobei das Herzmodell Magnetlokalisierungsdaten aufweist, die gemessen werden, indem der mindestens eine magnetische Lokalisierungssensor (70) verwendet wird; und
Lokalisieren des elektrophysiologischen Katheters (60), der in das Herz des Patienten eingeführt wird, nachdem der Katheter (13) aus dem Herz des Patienten entfernt worden ist, an einem Anfangsort innerhalb eines Koordinatensystems, das dem Herzmodell gemeinsam ist, indem der mindestens eine magnetische Lokalisierungssensor (70) des elektrophysiologischen Katheters (60) verwendet wird zum Durchführen der elektrophysiologischen Prozedur unter Verwendung des elektrophysiologischen Katheters (60).

2. System nach Anspruch 1, bei dem die elektrophysiologische Prozedur eine elektrophysiologische Abbildungsprozedur ist.

3. System nach Anspruch 1, bei dem der elektrophysiologische Katheter (60) einen elektrophysiologischen Mehrelektroden-Abbildungskatheter aufweist.

4. System nach Anspruch 3, bei dem der elektrophysiologische Katheter (60) einen kontaktlosen elektrophysiologischen Abbildungskatheter aufweist.

5. System nach Anspruch 1, bei dem die elektrophysiologische Prozedur eine Ablationsprozedur aufweist.

6. System nach Anspruch 1, bei dem der eine oder die mehreren Prozessoren (28) ferner angepasst sind zum:
Überwachen einer Bewegung des elektrophysiologischen Katheters (60) von dem Anfangsort zu einem nachfolgenden Ort; und
Neulokalisieren des elektrophysiologischen Katheters (60) innerhalb des Koordinatensystems, das dem Herzmodell gemeinsam ist, indem der mindestens eine magnetische Lokalisierungssensor (70) des elektrophysiologischen Katheters (60) verwendet wird.

7. System nach Anspruch 1, bei dem das Lokalisieren des elektrophysiologischen Katheters (60) innerhalb eines Koordinaten-Systems, das dem Herzmodell gemeinsam ist, unter Verwendung des mindestens einen magnetischen Lokalisierungssensors (70) des elektrophysiologischen Katheters (60), ferner ein Bestimmen eines Drehwinkels des mindestens einen magnetischen Lokalisierungssensors (70) des elektrophysiologischen Katheters (60) aufweist.

8. System nach Anspruch 7, bei dem der elektrophysiologische Katheter (60) mindestens eine röntgendichte Markierung (70) aufweist, und bei dem das Bestimmen des Drehwinkels des mindestens einen magnetischen Lokalisierungssensors (70) des elektrophysiologischen Katheters (60) ein Verwenden der mindestens einen röntgendichten Markierung (74) aufweist, um den Drehwinkel des mindestens einen magnetischen Lokalisierungssensors (70) des elektrophysiologischen Katheters (60) zu bestimmen.

9. System nach Anspruch 7, bei dem der elektrophysiologische Katheter (60) einen elektrophysiologischen Mehrelektroden-Abbildungskatheter aufweist, und bei dem das Bestimmen des Drehwinkels des mindestens einen magnetischen Lokalisierungssensors (70) des elektrophysiologischen Katheters (60) ein Verwenden der mehreren Elektroden des elektrophysiologischen Abbildungskatheters aufweist, um den Drehwinkel des mindestens einen magnetischen Lokalisierungssensors (70) des elektrophysiologischen Katheters (60) zu bestimmen.

10. System nach Anspruch 1, bei dem der mindestens eine magnetische Lokalisierungssensor (70) des elektrophysiologischen Katheters (60) auf einem Schaft des elektrophysiologischen Katheters (60) positioniert ist.

11. System nach Anspruch 1, bei dem der eine oder die mehreren Prozessoren (28) ferner angepasst sind zum Kompensieren einer Bewegung des elektrophysiologischen Katheters (60) während der elektrophysiologischen Prozedur unter Verwendung des mindestens einen magnetischen Lokalisierungssensors (70) des elektrophysiologischen Katheters (60).

12. System nach Anspruch 1, bei dem die elektrophysiologische Prozedur eine diagnostische Prozedur ist.

13. System nach Anspruch 1, bei dem die elektrophysiologische Prozedur eine therapeutische Prozedur ist.

## Revendications

1. Système pour effectuer une procédure électrophysiologique cardiaque, comprenant :
un cathéter (13) comprenant au moins un capteur de localisation magnétique (70) ;
un cathéter électrophysiologique (60) comprenant au moins un capteur de localisation magnétique (70) ; et
un ou plusieurs processeurs (28) adaptés pour :
générer un modèle cardiaque en utilisant le cathéter (13) inséré dans le cœur du patient, dans lequel le modèle cardiaque comprend des données de localisation magnétique mesurées en utilisant ledit au moins un capteur de localisation magnétique (70) ; et
localiser le cathéter électrophysiologique (60) qui est inséré dans le cœur du patient après que le cathéter (13) a été retiré du cœur du patient à un emplacement initial dans un système de coordonnées commun au modèle cardiaque en utilisant ledit au moins un capteur de localisation magnétique (70) du cathéter électrophysiologique (60) pour effectuer la procédure électrophysiologique en utilisant le cathéter électrophysiologique (60).

2. Système selon la revendication 1, dans lequel la procédure électrophysiologique comprend une procédure de cartographie électrophysiologique.

3. Système selon la revendication 1, dans lequel le cathéter électrophysiologique (60) comprend un cathéter de cartographie électrophysiologique à électrodes multiples.

4. Système selon la revendication 3, dans lequel le cathéter électrophysiologique (60) comprend un cathéter de cartographie électrophysiologique sans contact.

5. Système selon la revendication 1, dans lequel la procédure électrophysiologique comprend une procédure d'ablation.

6. Système selon la revendication 1, dans lequel le ou les processeurs (28) sont en outre adaptés pour :
surveiller le déplacement du cathéter électrophysiologique (60) de l'emplacement initial à un emplacement ultérieur ; et
relocaliser le cathéter électrophysiologique (60) dans le système de coordonnées commun au modèle cardiaque en utilisant ledit au moins un capteur de localisation magnétique (70) du cathéter électrophysiologique (60).

7. Système selon la revendication 1, dans lequel la localisation du cathéter électrophysiologique (60) dans un système de coordonnées commun au modèle cardiaque en utilisant ledit au moins un capteur de localisation magnétique (70) du cathéter électrophysiologique (60) comprend en outre une détermination d'un angle de rotation dudit au moins un capteur de localisation magnétique (70) du cathéter électrophysiologique (60).

8. Système selon la revendication 7, dans lequel le cathéter électrophysiologique (60) comprend au moins un marqueur radio-opaque (74), et la détermination de l'angle de rotation dudit au moins un capteur de localisation magnétique (70) du cathéter électrophysiologique (60) comprend l'utilisation dudit au moins un marqueur radio-opaque (74) pour déterminer l'angle de rotation dudit au moins un capteur de localisation magnétique (70) du cathéter électrophysiologique (60).

9. Système selon la revendication 7, dans lequel le cathéter électrophysiologique (60) comprend un cathéter de cartographie électrophysiologique à électrodes multiples, et dans lequel la détermination de l'angle de rotation dudit au moins un capteur de localisation magnétique (70) du cathéter électrophysiologique (60) comprend une utilisation des électrodes multiples du cathéter de cartographie électrophysiologique pour déterminer l'angle de rotation dudit au moins un capteur de localisation magnétique (70) du cathéter électrophysiologique (60).

10. Système selon la revendication 1, dans lequel ledit au moins un capteur de localisation magnétique (70) du cathéter électrophysiologique (60) est positionné sur une tige du cathéter électrophysiologique (60).

11. Système selon la revendication 1, dans lequel le ou les processeurs (28) sont en outre adaptés pour compenser le mouvement du cathéter électrophysiologique (60) pendant la procédure électrophysiologique en utilisant ledit au moins un capteur de localisation magnétique (70) du cathéter électrophysiologique (60).

12. Système selon la revendication 1, dans lequel la procédure électrophysiologique comprend une procédure de diagnostic.

13. Système selon la revendication 1, dans lequel la procédure électrophysiologique comprend une procédure thérapeutique.
